# DEMANDE DE BREVET EUROPEEN

(11) **EP 1 764 063 A2**
(43) Date de publication de la demande: **21.03.2007**
(21) Numéro de dépôt: 06077170.6
(22) Date de dépôt: 04.03.2003
(51) Int. Cl.: A61F 2/00, A61B 17/04

(54) **Bandelette pour le traitement de l'incontinence urinaire d'effort chey la femme**

(30) Priorité: 08.03.2002 FR 0203020
(62) Demande divisionnaire de: 03356038.4
(71) Demandeur: CL MEDICAL, 69110 Sainte Foy Les Lyon (FR)
(72) Inventeur: Goria, Vincent, F-69005 Lyon (FR); Grisard-Anaf, Maryelle, F-69003 Lyon (FR); Frobert, Jean-Luc, F-69006 Lyon (FR); Vautherin, Renaud, F-69420 Condrieu (FR); Lienhart, Jean, F-38200 Vienne (FR)
(74) Mandataire: Grand, Guillaume

(57) **Abrégé**

La bandelette (4), destinée à être implantée dans l'abdomen de la femme de façon à réaliser un soutien de l'urètre, comprend une première extrémité (9) et une deuxième extrémité pourvues de moyens de solidarisation analogues (12) destinés à la fixation de la bandelette sur la partie de préhension (7) ou sur l'extrémité pointue d'une aiguille (2). Pour notamment faciliter l'accrochage de la bandelette à une aiguille, les moyens de solidarisation analogues (12) se présentent sous la forme d'un manchon creux rigide (16A) prévu pour s'encliqueter autour de l'aiguille.

## Description

La présente invention concerne un dispositif chirurgical pour le traitement de l'incontinence urinaire d'effort chez la femme.

La présente invention concerne en particulier une bandelette destinée à être implantée dans le corps de la femme, de façon à réaliser un soutien de l'urètre pour ce même traitement de l'incontinence.

La présente invention se rapporte à une méthode de traitement, de correction et/ou d'atténuation de l'incontinence urinaire d'effort chez la femme.

Les problèmes d'incontinence urinaire sont actuellement traités au cours d'une intervention chirurgicale par l'implantation d'une bandelette réalisant un soutien de l'urètre. La bandelette est accrochée à chacune de ses extrémités à deux aiguilles perforantes permettant le passage à travers les tissus.

La voie d'implantation la plus utilisée est la voie vaginale, appelée également voie « basse ». Les deux aiguilles passent par une incision vaginale sous l'urètre, remontent de part et d'autre de l'urètre et de la vessie, puis traversent l'abdomen derrière la symphyse pubienne et ressortent au niveau abdominal sus-pubien. La bandelette suit le même trajet et vient s'ancrer sous tension dans la paroi abdominale.

De nombreux chirurgiens s'intéressent maintenant à des nouvelles voies. Tout d'abord, il existe la voie abdominale, également appelée voie « haute », c'est-à-dire avec un passage par le même trajet que la voie vaginale, mais en sens inverse de haut en bas. Il existe également la voie dite « latérale », c'est-à-dire avec un passage par les trous obturateurs droite et gauche de l'os iliaque. Les avantages de ces nouvelles voies sont nombreux. Le passage est plus simple, plus sécurisé. Il y a ainsi une limitation très forte des cas de morbidité décrits dans la littérature scientifique.

WO-00/74594 décrit un dispositif chirurgical pour le traitement de l'incontinence urinaire d'effort chez la femme, utilisable pour la voie « basse » et comportant une bandelette destinée à être implantée dans le corps de la patiente. Cette bandelette est pourvue à chacune de ses extrémités de moyens de solidarisation à une aiguille du dispositif. Plus précisément, ce document envisage l'utilisation soit d'un premier type de bandelette dont chaque extrémité est solidarisée à demeure, par de la colle ou par une enveloppe rétractable à chaud, au niveau d'une zone de préhension de l'aiguille, soit d'un second type de bandelette dont chaque extrémité est prévue pour être rapportée, éventuellement de façon amovible, à l'extrémité pointue de l'aiguille par coopération de languettes solidaires de la bandelette encliquetées dans des orifices correspondants de l'aiguille.

Un chirurgien qui utilise le dispositif du document WO-00/74594 doit donc choisir avant l'intervention chirurgicale le type de bandelette adéquat selon notamment qu'il souhaite pratiquer une intervention pas voie basse, voie haute ou voie latérale comme expliqué ci-dessus. Cela implique donc que la structure hospitalière où se déroule l'intervention possède les différents types de bandelette envisageables, dans des stocks suffisants, et s'assure que, pour chaque type de bandelette, des aiguilles correspondantes sont disponibles. L'utilisation et la gestion des stocks de ce dispositif sont donc complexes, engendrent des sur-coûts et compliquent le travail du chirurgien.

Le but de l'invention est la mise au point d'un instrument chirurgical simple d'utilisation qui puisse être très facilement adapté à la voie « basse », « haute » ou « latérale » choisie, sans compliquer le travail du chirurgien, notamment en lui facilitant la fixation des bandelettes sur des aiguilles, ni obliger à prévoir des stocks de matériel importants.

A cet effet, l'invention a pour objet une bandelette telle que définie dans la revendication 1.

L'accrochage de la bandelette se fait facilement et solidement. La ou les aiguilles sur lesquelles la bandelette se fixe sont spécialement conçues pour un passage très facile et un accrochage sécurisé avec la bandelette sous-urétrale quel que soit le sens de traction de la bandelette à l'intérieur de l'abdomen de la patiente. Le dispositif chirurgical comprenant la bandelette et cette ou ces aiguilles permet d'utiliser indifféremment, et avec la même facilité, les trois voies répertoriées.

Le chirurgien peut choisir la méthode la plus adaptée et la plus sécurisée, et celle qui s'adaptera le mieux au tableau clinique de la patiente. Avec ces moyens de solidarisation particulièrement souples, le chirurgien peut décider au dernier moment de la technique qu'il va employer par la voie « basse », « haute » ou « latérale ». Il peut ainsi très facilement fixer la bandelette vers la partie de préhension pour la voie « basse ». Il peut ainsi très facilement fixer la bandelette vers la pointe pour les voies « haute » ou « latérale ».

Le chirurgien limite le nombre de manipulations durant l'intervention chirurgicale. L'usage très simple du dispositif chirurgical contribue ainsi à la réduction des risques peropératoires. De plus, les moyens de solidarisation retenue sont peu coûteux.

D'autres caractéristiques avantageuses de la bandelette sont définies dans les revendications dépendantes 2 à 9.

L'invention sera bien comprise et ses divers avantages et différentes caractéristiques ressortiront mieux lors de la description suivante, des exemples non limitatifs de réalisation, en référence aux dessins schématiques annexés, dans lesquels :
- la figure 1 représente une vue en perspective d'un dispositif chirurgical avec deux aiguilles et une bandelette montée pour une intervention par la voie « basse » ;
- la figure 2 représente une vue en plan partielle d'un dispositif chirurgical avec une aiguille et une bandelette montée pour une intervention par la voie « basse » ;
- la figure 3 représente une vue en plan partielle de la pointe de l'aiguille ;
- la figure 4 représente une vue en plan partielle de la partie de préhension de l'aiguille ;
- la figure 5 représente une vue en coupe longitudinale partielle de l'aiguille, du manchon dans une première forme de réalisation et de la bandelette ;
- la figure 6 représente une vue en plan d'une variante de réalisation de l'aiguille ;
- la figure 7 représente une vue en perspective du manchon dans une deuxième forme de réalisation ;
- la figure 8 représente une vue en coupe longitudinale du manchon de la figure 7 ;
- la figure 9 représente une vue partielle en perspective de la région abdominale d'une patiente, montrant le trajet de la bandelette implantée par la voie « basse » ;
- la figure 10 représente une vue partielle en perspective de la région abdominale d'une patiente, montrant le trajet de la bandelette implantée par la voie « haute » ;
- la figure 11 représente une vue partielle en perspective de la région abdominale d'une patiente, montrant le trajet de la bandelette implantée par la voie « latérale » ;
- Les figures 12 et 13 sont des vues analogues aux figures 7 et 8, illustrant une troisième forme de réalisation du manchon ;
- La figure 14 est une vue analogue à la figure 6, d'une autre variante de l'aiguille ;
- la figure 15 est une vue en perspective d'une poignée de manipulation du dispositif, adaptée pour l'aiguille de la figure 14 ;
- la figure 16 est une vue en perspective du dispositif comportant l'aiguille de la figure 14, le manchon des figures 12 et 13 et la poignée de la figure 15 ; et
- la figure 17 est une vue schématique en plan et à grande échelle de la structure du tissu formant la bandelette du dispositif.

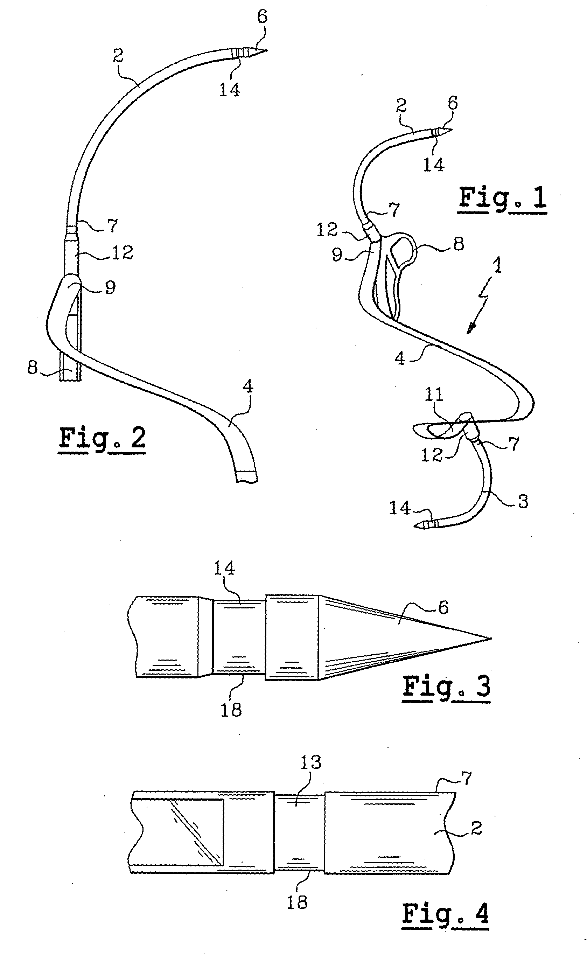

Sur les figures 1 à 8 et 12 à 15 est représenté un dispositif chirurgical 1 destiné au traitement de l'incontinence urinaire d'effort chez une patiente. Le dispositif 1 comprend deux aiguilles 2, 3 reliées entres-elles par une bandelette 4. Par son implantation spécifique dans l'abdomen de la patiente devant être traitée, la bandelette 4 va permettre un soutien de l'urètre. La bandelette 4 est réalisée en un tissu à base de monofilament de polypropylène.

Les aiguilles 2, 3 sont en acier chirurgical et possèdent un diamètre sensiblement constant. Les aiguilles 2, 3 présentent chacune une extrémité pointue 6 et une partie de base 7 plus particulièrement affectée à leur préhension. Une poignée amovible et facultative 8, avec par exemple une forme cardioïde, vient se fixer par clavetage (ou également par vissage ou par encliquetage) à la base de l'aiguille 2 vers la partie de base 7.

Une première extrémité 9 de la bandelette 4 va se solidariser à la première aiguille 2 et une seconde extrémité 11 de la bandelette 4 va se solidariser à la seconde aiguille 3.

Pour ce faire, la bandelette 4 comprend à sa première extrémité 9 des moyens de solidarisation 12 assurant sa fixation à la première aiguille 2. Et la bandelette 4 comprend à sa seconde extrémité 11 des moyens de solidarisation assurant sa fixation à la seconde aiguille 3. Les moyens de solidarisation de la seconde extrémité 11 de la bandelette 4 n'ont pas été représentés, car ils sont strictement analogues aux moyens de solidarisation de la première extrémité 9.

Pour réaliser les trajets d'intervention par la voie « basse », l'aiguille 2 comprend des premiers moyens de solidarisation 13, qui sont complémentaires des moyens de solidarisation 12 de la bandelette 4 et qui sont disposés au niveau de la partie de préhension 7. Pour réaliser les trajets d'intervention par les voies « haute » et « latérale », l'aiguille 2 comprend des deuxièmes moyens de solidarisation 14, qui sont complémentaires des moyens de solidarisation 12 de la bandelette 4 et qui sont disposés au niveau de l'extrémité pointue 6. Afin de pouvoir utiliser la même bandelette 4, les premiers moyens de solidarisation 13 et les deuxièmes moyens de solidarisation 14 de l'aiguille 2 sont strictement analogues.

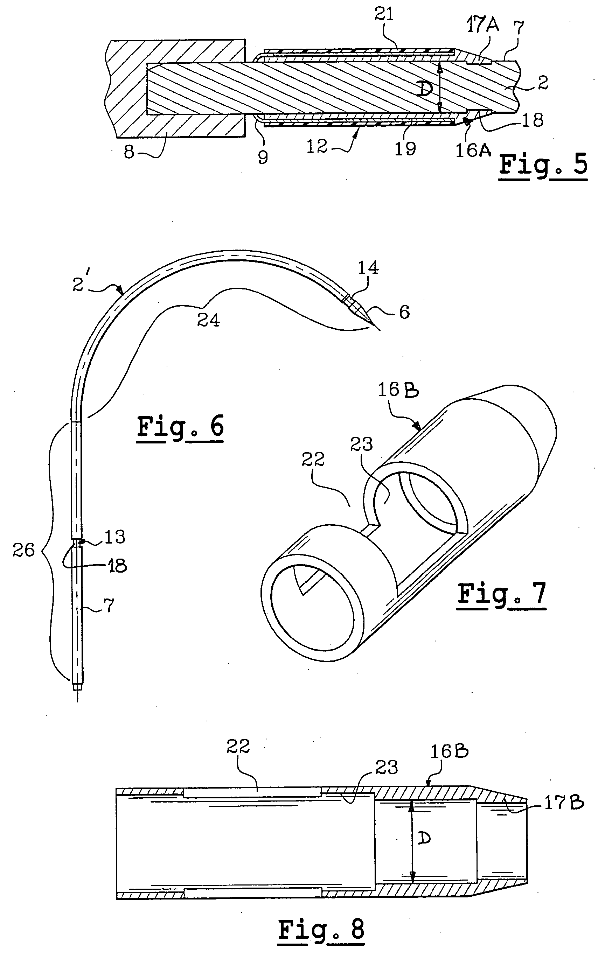

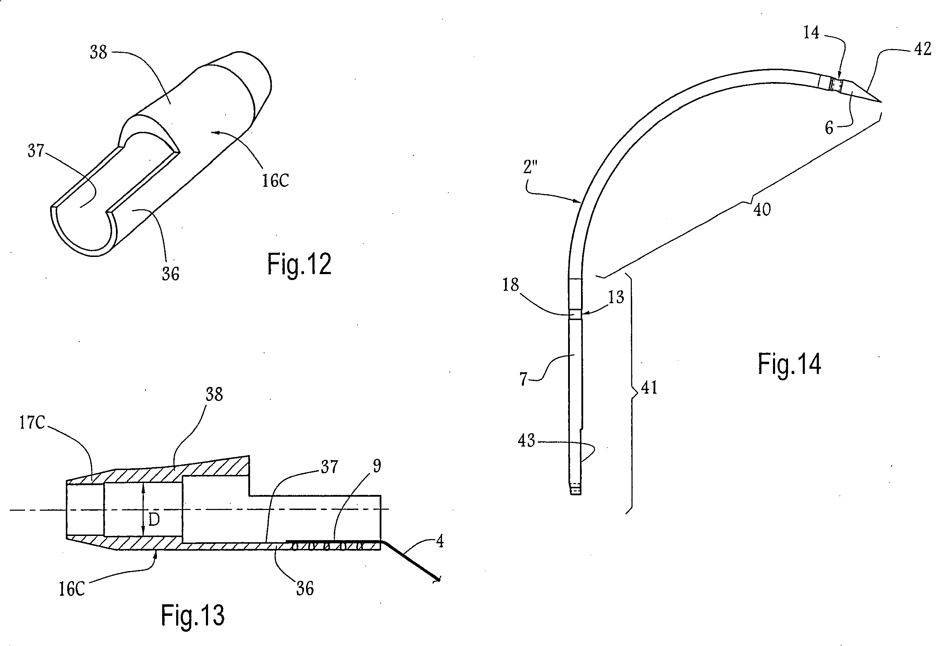

Pour le dispositif représenté, les moyens de solidarisation 12 de la bandelette 4 se présentent sous la forme d'un manchon creux, noté 16A sur la figure 5 qui correspond à une première forme de réalisation, 16B sur les figures 7 et 8 qui correspondent à une seconde forme de réalisation, et 16C sur les figures 12 et 13 qui correspondent à une troisième forme de réalisation. Ce manchon 16A, 16B ou 16C est en un matériau polymère rigide et vient s'enfiler autour de l'aiguille 2. De ce fait, le diamètre intérieur D de la partie courante du manchon 16 est sensiblement identique ou à peine supérieur au diamètre de l'aiguille 2. Le manchon 16A, 16B ou 16C comprend à l'une de ses extrémités une partie formant rétreint ou nervure circulaire interne 17A, 17B, 17C avec ainsi un diamètre intérieur plus faible que le diamètre intérieur du reste du manchon. Au niveau de l'extrémité avec la partie formant rétreint 17A, 17B, 17C, le manchon se termine en tronc de cône. Ceci est utilisé pour faciliter la progression de l'aiguille 2 avec sa bandelette 4 dans les tissus de l'abdomen.

La partie formant rétreint 17A, 17B, 17C va coopérer avec les premiers moyens de solidarisation 13 ou les deuxièmes moyens de solidarisation 14 de l'aiguille 2. Pour ce faire, les premiers moyens de solidarisation 13 ou les deuxièmes moyens de solidarisation 14 de l'aiguille 2 sont sous la forme d'une rainure circonférencielle 18 ménagée dans le corps de l'aiguille 2. Ainsi, la nervure circulaire interne 17A, 17B et 17C du manchon 16 va pouvoir s'encliqueter très facilement dans la rainure circulaire 18 de l'extrémité pointue 6 ou de la partie de base 7.

Dans la première forme de réalisation (voir Figure 5), le manchon 16A va comprendre une zone d'attache circonférencielle externe 19 autour de laquelle va pouvoir se fixer l'extrémité 9 de la bandelette 4. Cette extrémité 9 peut être collée sur un secteur de la zone d'attache 19. Une gaine souple thermorétractable 21 vient recouvrir et immobiliser fermement l'extrémité 9 de la bandelette 4.

Dans la deuxième forme de réalisation (voir Figures 7 et 8), une ouverture ou fenêtre 22 est ménagée à travers la paroi du manchon creux 16B. Le manchon 16B va également présenter une zone d'attache circonférencielle interne 23 prévue entre la nervure circulaire interne 17B et la fenêtre 22. Cette zone d'attache circonférencielle interne 23 possède un diamètre intérieur supérieur au diamètre extérieur de l'aiguille 2. De ce fait, l'extrémité 9 de la bandelette 4 va pouvoir s'agencer et être mécaniquement coincée entre la zone d'attache circonférencielle interne 23 du manchon 16B et le corps de l'aiguille 2. Le reste de la bandelette 4 va sortir par la fenêtre 22.

Dans la troisième forme de réalisation (voir figures 12 et 13), la paroi du manchon creux 16C forme, dans sa partie d'extrémité opposée à la partie formant rétreint 17C, un semi-tube 36 de section transversale semi-circulaire. Cette partie semi-tubulaire 36 est par exemple obtenue à partir du manchon 16B des figures 7 et 8 pour lequel l'ouverture 22 est prolongée jusqu'à l'extrémité du manchon. La partie semi-tubulaire 36 possède un diamètre intérieur supérieur au diamètre extérieur de l'aiguille 2 et présente une zone d'attache circonférentielle interne 37 dans laquelle l'extrémité 9 ou 11 de la bandelette 4 peut être cousue de façon à solidariser le manchon à la bandelette, comme représenté sur la figure 13.

Le manchon 16C des figures 12 et 13 se distingue également des manchons 16A et 16B par la partie de paroi 38 située dans le prolongement de l'ouverture latérale formé par la partie semi-tubulaire 36. Cette partie de paroi 38 présente une surface extérieure sensiblement tronconique convergente vers l'extrémité 17C pour faciliter la progression de l'aiguille 2 munie de la bandelette dans les tissus abdominaux.

Dans une première variante de réalisation de l'aiguille représentée sur la figure 6, l'aiguille, notée 2' est conformée de manière différente. L'aiguille 2' présente une première portion 24 pratiquement recourbée en demi-cercle. L'aiguille 2 présente ensuite une deuxième portion totalement rectiligne 26 dans la continuité et d'un seul tenant avec la première portion 24 et de même diamètre que cette dernière.

Cette deuxième portion totalement rectiligne 26 forme la partie de préhension 7. De ce fait, une présence de la poignée 8 n'est plus forcément indispensable. Dans cette variante de réalisation, la rainure circulaire 18 des premiers moyens de solidarisation 13 de l'aiguille 2 est ménagée sensiblement au milieu de la portion rectiligne 26.

Dans une deuxième variante de l'aiguille, notée 2" et représentée sur la figure 14, l'aiguille 2'' présente une portion recourbée 40 et une portion totalement rectiligne 41, analogues aux portions 24 et 26 de l'aiguille 2'. L'extrémité pointue 6 de l'aiguille 2'' se distingue des extrémités pointues des aiguilles 2 et 2' par sa forme biseautée, une surface de biseau 42 étant formée du côté extrados de la portion recourbée 40. La pénétration et le guidage de l'extrémité 6 dans les tissus humains s'en trouvent facilités.

La portion rectiligne 41, qui forme la partie de préhension 7, porte la rainure circulaire 18 et présente dans sa partie d'extrémité libre un méplat latéral 43.

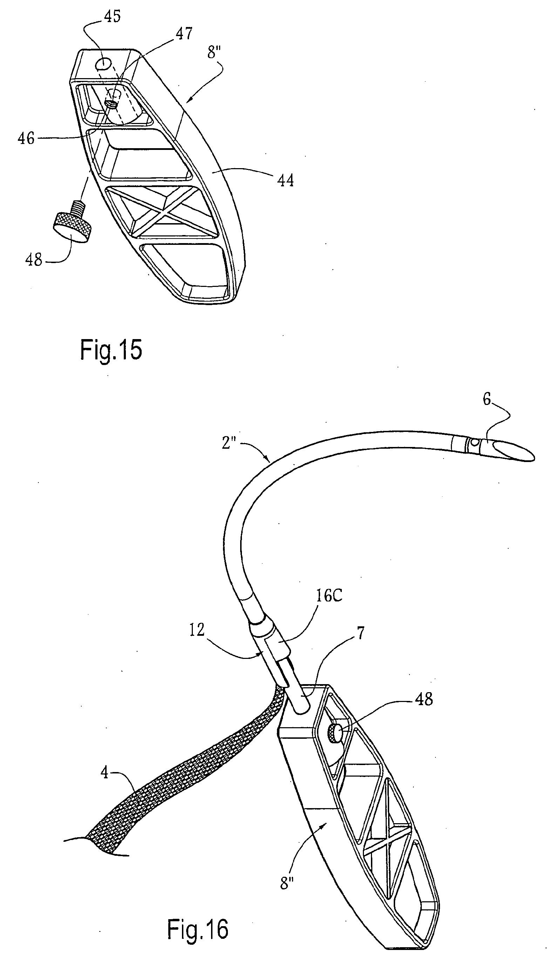

Sur la figure 15 est rerpésentée une variante de la poignée 8, notée 8", adaptée pour être montée et solidarisée à l'aiguille 2". Plus précisément, cette poignée 8" comporte un corps rigide plan 44, par exemple en plastique moulée, de contour latéral ovoïde et pourvu à une de ses extrémités d'un orifice 45 de réception de la partie de préhension 7 de l'aiguille 2. Cet orifice présente, en section transversale, un profil complémentaire de l'extrémité libre de la partie rectiligne 41. La coopération du méplat 43 et de cet orifice impose l'orientation et le blocage angulaires relatifs entre la poignée et l'aiguille de sorte qu'une des deux faces planes du corps 44 est dirigée dans la direction visée par la portion recourbée 40 de l'aiguille.

Pour assurer la retenue en translation de la poignée amovible 8" à l'aiguille 2", un trou 46 débouchant dans l'orifice 45 est formé dans le corps 44, suivant une direction transversale à cet orifice. Le trou 46 présente un taraudage interne 47 adapté pour recevoir une vis 48 de blocage de l'aiguille. En fin de vissage, l'extrémité pointue de la vis coopère, par exemple, avec une empreinte creusée dans le méplat 43.

Les différentes variantes décrites ci-dessus de l'aiguille, du manchon et de la poignée du dispositif chirurgical 1 peuvent bien entendu être combinées entre elles. A titre d'exemple, sur la figure 16 est représenté un dispositif selon l'invention comportant l'aiguille 2", le manchon 16C et la poignée 8".

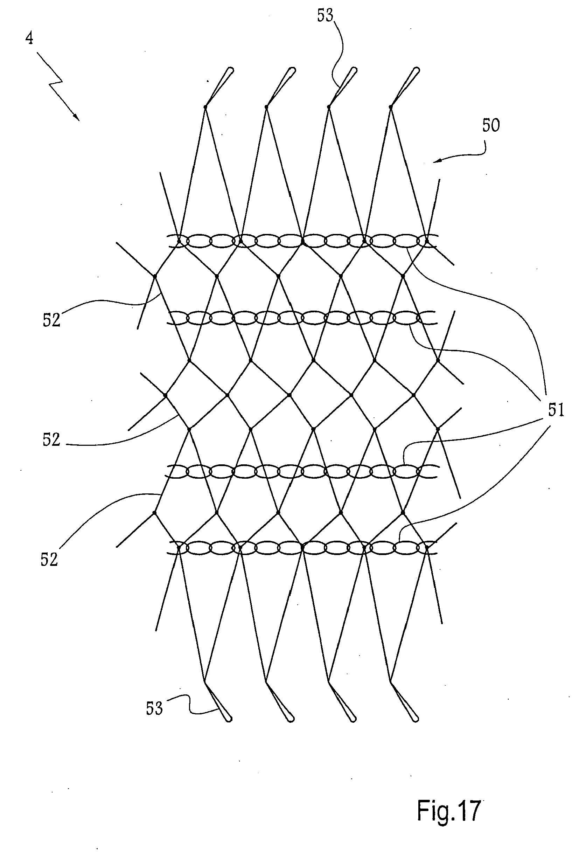

Sur la figure 17 est représentée le corps de la bandelette 4. Comme indiqué précédemment, le tissu constituant la bandelette est à base de monofilaments de propylène, tricotés de façon à former une structure 50 qui comporte essentiellement :
- quatre chaînettes longitudinales tricotées 51 parallèles entre elles et formant l'armature de la structure ;
- des treillis intermédiaires 52 reliant deux à deux les chaînettes 50, et
- deux franges latérales 53 constituées de filaments qui s'étendent transversalement au reste de la bandelette et destinés à faciliter l'accrochage des tissus lorsque la bandelette est implantée dans le corps humain.

Cette structure 50 présente l'avantage de présenter une bonne tenue à la traction et un risque très faible de démaillage.

Trois utilisations du dispositif chirurgical selon l'invention décrit ci-dessus vont être décrites ci-après.

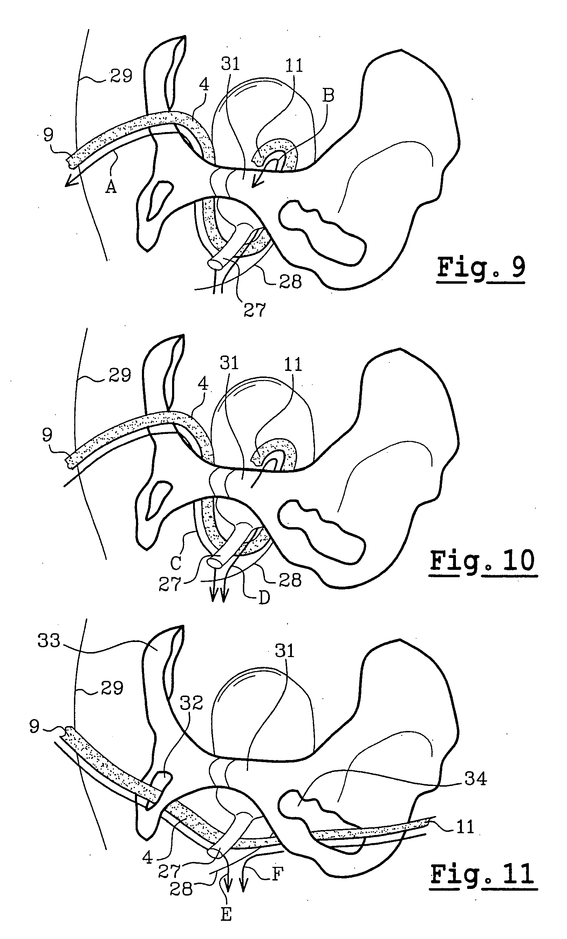

La première méthode de traitement, de correction et/ou d'atténuation de l'incontinence urinaire d'effort chez une femme, dite par la voie « basse » (voir Figure 9), comprend les étapes consistant à :
(a) choisir une bandelette 4 destinée à être implantée dans l'abdomen de la femme de façon à réaliser un soutien de l'urètre 27, une première extrémité 9 et une deuxième extrémité 11 de la bandelette 4 pouvant être respectivement solidarisée à deux aiguilles 2, 3, la bandelette 4 comprenant à sa première extrémité 9 et à sa deuxième extrémité 11 des moyens de solidarisation analogues 12 destinés à sa fixation aux deux aiguilles 2, 3 ;
(b) choisir les deux aiguilles 2, 3 présentant chacune une extrémité pointue 6 et une partie de préhension 7, les deux aiguilles 2, 3 étant dimensionnées pour s'étendre de la surface intérieure de la paroi vaginale 28 jusqu'à l'extérieur de la paroi abdominale 29, les deux aiguilles 2, 3 comprenant chacune des moyens de solidarisation analogues 13, 14 complémentaires des moyens de solidarisation analogues 12 de la bandelette 4, disposés à la fois au niveau de sa partie de préhension 7 et vers son extrémité pointue 6 ;
(c) connecter les moyens de solidarisation 12 de la première extrémité 9 de la bandelette 4 aux moyens de solidarisation complémentaires 13 de la première aiguille 2 disposés au niveau de sa partie de préhension 7 ;
(d) connecter les moyens de solidarisation 12 de la deuxième extrémité 11 de la bandelette 4 aux moyens de solidarisation complémentaires 13 de la deuxième aiguille 3 disposés au niveau de sa partie de préhension 7 ;
(e) faire passer la première aiguille 2 et la première extrémité 9 de la bandelette 4 d'un côté de l'urètre 27 à partir d'une incision de la paroi vaginale 28 à l'intérieur de l'abdomen et derrière la symphyse pubienne 31 pour faire ressortir la première aiguille 2 et la première extrémité 9 de la bandelette 4 au niveau d'une première incision de la paroi abdominale 29 (voir Flèche A en Figure 9) ;
(f) soit déconnecter les moyens de solidarisation 12 de la première extrémité 9 de la bandelette 4 des moyens de solidarisation complémentaires 13 de la première aiguille 2, soit couper la bandelette 4 au ras de ses moyens de solidarisation 12 ;
(g) faire passer la deuxième aiguille 3 et la deuxième extrémité 11 de la bandelette 4 de l'autre côté de l'urètre 27 à partir de l'incision de la paroi vaginale 28 à l'intérieur de l'abdomen et derrière la symphyse pubienne 31) pour faire ressortir la deuxième aiguille 3 et la deuxième extrémité 11 de la bandelette 4 au niveau d'une seconde incision de la paroi abdominale 29 (voir Flèche B en Figure 9) ;
(h) soit déconnecter les moyens de solidarisation 12 de la deuxième extrémité 11 de la bandelette 4 des moyens de solidarisation complémentaires 13 de la deuxième aiguille 3, soit couper la bandelette 4 au ras de ses moyens de solidarisation 12 ;
(i) tirer sur la première extrémité 9 et sur la deuxième extrémité 11 de la bandelette 4 et ajuster la tension, de façon à réaliser un soutien de l'urètre 27, puis laisser la bandelette 4 dans l'abdomen.

On notera que l'ordre des étapes (c) (d) (e) (f) et (g) peut être inversé. Le chirurgien peut soit connecter la bandelette 4 à l'aiguille 2, 3 et passer son aiguille 2, 3, soit passer l'aiguille 2, 3, faire tout le trajet, et seulement quand le trajet est bon, connecter la bandelette 4. Mais, il doit disposer de suffisamment d'espace libre pour pouvoir effectuer la connexion. Si l'aiguille 2, 3 est trop enfoncée à travers l'abdomen, cela ne sera plus possible.

La deuxième méthode de traitement, de correction et/ou d'atténuation de l'incontinence urinaire d'effort chez une femme, dite par la voie « haute » (voir Figure 10), comprend les étapes consistant à :
(a) choisir une bandelette 4 destinée à être implantée dans l'abdomen de la femme de façon à réaliser un soutien de l'urètre 27, une première extrémité 9 et une deuxième extrémité 11 de la bandelette 4 pouvant être respectivement solidarisée à deux aiguilles 2, 3, la bandelette 4 comprenant à sa première extrémité 9 et à sa deuxième extrémité 11 des moyens de solidarisation analogues 12 destinés à sa fixation aux deux aiguilles 2, 3 ;
(b) choisir deux aiguilles 2, 3 présentant chacune une extrémité pointue 6 et une partie de préhension 7, les deux aiguilles 2, 3 étant dimensionnées pour s'étendre de la surface intérieure de la paroi vaginale 28 jusqu'à l'extérieur de la paroi abdominale 29, les deux aiguilles 2, 3 comprenant chacune des moyens de solidarisation analogues 13, 14 complémentaires des moyens de solidarisation analogues 12 de la bandelette 4, disposés à la fois au niveau de sa partie de préhension 7 et vers son extrémité pointue 6 ;
(c) faire passer la première aiguille 2 à partir d'une première incision de la paroi abdominale 29 à l'intérieur de l'abdomen derrière la symphyse pubienne 31 d'un côté de l'urètre 27 jusqu'à une incision de la paroi vaginale 28 (voir Flèche C en Figure 10) ;
(d) connecter les moyens de solidarisation 12 de la première extrémité 9 de la bandelette 4 aux moyens de solidarisation complémentaires 14 de la première aiguille 2 disposés au niveau de son extrémité pointue 6 ;
(e) tracter la première extrémité 9 de la bandelette 4 avec la première aiguille 2 en effectuant le trajet inverse à l'étape (c) ;
(f) soit déconnecter les moyens de solidarisation 12 de la première extrémité 9 de la bandelette 4 des moyens de solidarisation complémentaires 14 de la première aiguille 2, soit couper la bandelette 4 au ras de ses moyens de solidarisation 12 ;
(g) faire passer la deuxième aiguille 3 à partir d'une seconde incision de la paroi abdominale 29 à l'intérieur de l'abdomen derrière la symphyse pubienne 31 de l'autre côté de l'urètre 27 jusqu'à l'incision de la paroi vaginale 28 (voir Flèche D en Figure 10) ;
(h) connecter les moyens de solidarisation 12 de la deuxième extrémité 11 de la bandelette 4 aux moyens de solidarisation complémentaires 14 de la deuxième aiguille 3 disposés au niveau de son extrémité pointue 6 ;
(i) tracter la deuxième extrémité 11 de la bandelette 4 avec la deuxième aiguille 3 en effectuant le trajet inverse à l'étape (g) ;
(j) soit déconnecter les moyens de solidarisation 12 de la deuxième extrémité 11 de la bandelette 4 des moyens de solidarisation complémentaires 14 de la deuxième aiguille 3, soit couper la bandelette 4 au ras de ses moyens de solidarisation 12 ;
(k) tirer sur la première extrémité 9 et sur la deuxième extrémité 11 de la bandelette 4 et ajuster la tension, de façon à réaliser un soutien de l'urètre 27, puis laisser la bandelette 4 dans l'abdomen.

La troisième méthode de traitement, de correction et/ou d'atténuation de l'incontinence urinaire d'effort chez une femme, dite par la voie « latérale » (voir Figure 11), comprend les étapes consistant à :
(a) choisir une bandelette 4 destinée à être implantée dans l'abdomen de la femme de façon à réaliser un soutien de l'urètre 27, une première extrémité 9 et une deuxième extrémité 11 de la bandelette 4 pouvant être respectivement solidarisée à deux aiguilles 2, 3, la bandelette 4 comprenant à sa première extrémité 9 et à sa deuxième extrémité 11 des moyens de solidarisation analogues 12 destinés à sa fixation aux deux aiguilles 2, 3 ;
(b) choisir deux aiguilles 2, 3 présentant chacune une extrémité pointue 6 et une partie de préhension 7, les deux aiguilles 2, 3 étant dimensionnées pour s'étendre de la surface intérieure de la paroi vaginale 28 jusqu'à l'extérieur de la paroi abdominale 29, les deux aiguilles 2, 3 comprenant chacune des moyens de solidarisation analogues 14 complémentaires des moyens de solidarisation analogues 12 de la bandelette 4, disposés à la fois au niveau de sa partie de préhension 7 et vers son extrémité pointue 6 ;
(c) faire passer la première aiguille 2 à partir d'une première incision de la paroi de l'aine à l'intérieur de l'abdomen par l'un des trous obturateurs 32 de l'os iliaque 33 d'un côté de l'urètre 27 jusqu'à une incision de la paroi vaginale 28 (voir Flèche E en Figure 11) ;
(d) connecter les moyens de solidarisation 12 de la première extrémité 9 de la bandelette 4 aux moyens de solidarisation complémentaires 14 de la première aiguille 2 disposés au niveau de son extrémité pointue 6 ;
(e) tracter la première extrémité 9 de la bandelette 4 avec la première aiguille 2 en effectuant le trajet inverse à l'étape (c) ;
(f) soit déconnecter les moyens de solidarisation 12 de la première extrémité 9 de la bandelette 4 des moyens de solidarisation complémentaires 14 de la première aiguille 2, soit couper la bandelette 4 au ras de ses moyens de solidarisation 12 ;
(g) faire passer la deuxième aiguille 3 à partir d'une seconde incision de la paroi de l'aine à l'intérieur de l'abdomen par l'autre des trous obturateurs 34 de l'os iliaque 33 de l'autre côté de l'urètre 27 jusqu'à l'incision de la paroi vaginale 28 (voir Flèche F en Figure 11) ;
(h) connecter les moyens de solidarisation 12 de la deuxième extrémité 11 de la bandelette 4 aux moyens de solidarisation complémentaires 14 de la deuxième aiguille 3 disposés au niveau de son extrémité pointue 6 ;
(i) tracter la deuxième extrémité 11 de la bandelette 4 avec la deuxième aiguille 3 en effectuant le trajet inverse à l'étape (g) ;
(j) soit déconnecter les moyens de solidarisation 12 de la deuxième extrémité 11 de la bandelette 4 des moyens de solidarisation complémentaires 14 de la deuxième aiguille (3), soit couper la bandelette 4 au ras de ses moyens de solidarisation 12 ;
(k) tirer sur la première extrémité 9 et sur la deuxième extrémité 11 de la bandelette 4 et ajuster la tension, de façon à réaliser un soutien de l'urètre 27, puis laisser la bandelette 4 dans l'abdomen.

En variante à cette méthode par voie « latérale », le sens de déplacement des aiguilles 2 et 3 peut être inversé, c'est-à-dire que chaque aiguille est passée depuis une incision de la paroi vaginale à une incision de la paroi de l'aine en passant par l'un des trous obturateurs de l'os iliaque. En d'autres termes, les sens des flèches E et F de la figure 11 sont inversés.

On notera que, bien que les différentes méthodes de traitement ci-dessus ont été décrites en référence au premier mode de réalisation du dispositif représenté sur les figures 1 à 5, l'utilisation du dispositif selon les variantes décrites en regard des figures 6 à 8 et 12 à 15 est sensiblement analogue.

La présente invention n'est pas limitée aux modes de réalisation décrits et illustrés. De nombreuses modifications peuvent être réalisées, sans pour autant sortir du cadre défini par la portée du jeu de revendications.

## Revendications

1. Bandelette (4), destinée à être implantée dans l'abdomen d'une femme de façon à réaliser un soutien de l'urètre (27) pour le traitement de l'incontinence urinaire d'effort chez la femme, comprenant une première extrémité (9) et une deuxième extrémité (11) pourvues de moyens de solidarisation analogues (12) destinés à la fixation de la bandelette sur la partie de préhension (7) ou sur l'extrémité pointue (6) d'une aiguille (2, 3 ; 2' ; 2''), **caractérisée en ce que** les moyens de solidarisation analogues (12) de la bandelette (4) se présentent sous la forme d'un manchon creux rigide (16A ; 16B ; 16C) prévu pour s'encliqueter autour de l'aiguille (2 ; 2' ; 2'').

2. Bandelette selon la revendication 1, **caractérisée en ce qu'**elle est placée sur une surface (19) autour du manchon creux rigide (16A) et y est maintenue par une gaine thermorétractable (21).

3. Bandelette selon la revendication 1, **caractérisée en ce qu'**elle est maintenue entre le manchon creux rigide (16B) et l'aiguille (2 ; 2' ; 2''), et **en ce qu'**elle sort par une ouverture (22) ménagée dans le manchon creux rigide (16B).

4. Bandelette selon la revendication 1, **caractérisée en ce qu'**elle est cousue dans une paroi (36) du manchon (16C).

5. Bandelette selon la revendication 4, **caractérisée en ce que** ladite paroi (36) du manchon (16C) forme un semi-tube de section transversale semi-circulaire.

6. Bandelette selon l'une quelconque des revendications précédentes, **caractérisée en ce que** le manchon creux rigide (16A ; 16B ; 16C) comprend, à l'une de ses extrémités longitudinales, une partie formant rétreint (17A ; 17B ; 17C) apte à s'encliqueter dans une rainure de l'extrémité pointue (6) ou de la partie de préhension (7) d'une aiguille (2, 3 ; 2' ; 2'').

7. Bandelette selon la revendication 6, **caractérisée en ce qu'**au niveau de l'extrémité du manchon (16A ; 16B ; 16C) munie de la partie formant rétreint (17A ; 17B ; 17C), le manchon se termine en tronc de cône.

8. Bandelette selon l'une quelconque des revendications précédentes, **caractérisée en ce qu'**elle est constituée d'un tissu à base de monofilaments en polypropylène.

9. Bandelette selon la revendication 8, **caractérisée en ce que** les monofilaments en polypropylène du tissu sont tricotés de façon à former une armature constituée de chaînettes longitudinales (51) reliées deux à deux par des treillis (52).
